(19) Europäisches Patentamt
European Patent Office
Office européen des brevets

(11) Publication number : **0 471 453 A2**

(12) **EUROPEAN PATENT APPLICATION**

(21) Application number : **91306582.7**

(22) Date of filing : **19.07.91**

(51) Int. Cl.[5] : **C07K 7/52,** C07K 7/54, C12P 21/04, A61K 37/02, A61K 39/21

A request for correction of errors in the claims has been filed pursuant to Rule 88 EPC. A decision on the request will be taken during the proceedings before the Examining Division (Guidelines for Examination in the EPO, A-V, 2.2).

(30) Priority : **19.07.90 US 555112**

(43) Date of publication of application : **19.02.92 Bulletin 92/08**

(84) Designated Contracting States : **CH DE FR GB IT LI NL**

(71) Applicant : **MERCK & CO. INC.**
**126, East Lincoln Avenue P.O. Box 2000**
**Rahway New Jersey 07065-0900 (US)**

(72) Inventor : **Sugg, Elizabeth E.**
**4108 Livingstone Place**
**Durham, North Carolina 27707 (US)**
Inventor : **Dolan, Catherine A.**
**353 New Dover Road**
**Colonia, NJ 07067 (US)**
Inventor : **Bednarek, Maria A.**
**270 McFarlane Road, No. 167**
**Colonia, NJ 07067 (US)**
Inventor : **Tolman, Richard L.**
**29 Upper Warren Way**
**Warren, NJ 07059 (US)**
Inventor : **Christensen, Burton G.**
**770 Anderson Avenue**
**Cliffside Park, NJ 07010 (US)**

(74) Representative : **Barrett-Major, Julie Diane et al**
**Merck & Co., Inc. European Patent Department Terlings Park Eastwick Road**
**Harlow Essex CM20 2QR (GB)**

(54) **Cyclic HIV principal neutralizing determinant peptides.**

(57) Human Immunodeficiency Virus (HIV) Principal Neutralizing Determinant (PND) peptides, or peptides immunologically equivalent therewith, having the structure :

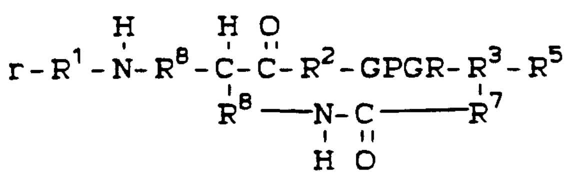

wherein the ring system is formed by ring closure through a stable amide bond, are useful as analytical tools, as reagents in ELISA assays, or as reagents for making covalent conjugate immunogens. Conjugates containing these stable, cyclic peptides are useful for raising mammalian anti-peptide, anti-HIV, or HIV-neutralizing immune responses, and a composition containing such a conjugate may be used as a vaccine to prevent HIV-Disease, including Acquired Immune Deficiency Syndrome, (AIDS), or AIDS related complex, (ARC), or as an immunogen for treating humans afflicted with HIV-Diseases, such as AIDS or ARC.

EP 0 471 453 A2

## BACKGROUND OF THE INVENTION

Human Immunodeficiency virus (HIV) Principal Neutralizing Determinant (PND) peptides have been described which are capable of raising an anti-HIV immune response in mammals. The hypervariable region of the AIDS virus envelope glycoprotein, gp120, between amino acids 296 and 341 [according to the numbering scheme of Ratner et al., Nature 313, 277 (1985)] contains the amino acid tetramer -GlyProGlyArg- (-GPGR-) in most of the HIV isolates identified to date. In addition, a cysteine residue is generally found within about 20 amino acids on either side of this tetramer. In at least one common isolate, HIV IIIB, it is known that these cysteines are disulfide bonded. Thus, the intermediate amino acids, herein referred to as loop amino acids, are forced into a cyclic structure, with the -GPGR- being exposed at the loop-tip [Javaherian et al., PNAS USA 86, 6768, (1989)].

Peptide based efforts aimed at the induction of HIV neutralizing immune responses in mammals have generally been limited to the use of linear or disulfide bonded cyclic peptides. The linear peptides have the limitation that the recipient immune system is exposed to an epitope which constantly changes its three dimensional conformation in solution, while reliance on disulfide bonding to limit the number of conformations assumable by the linear PNDs is not completely satisfactory as disulfides are labile. Breakage of the disulfides results in the linear peptide having the attendant problems described above.

In addition to the problems associated with linear or disulfide bonded peptides noted above, an additional problem is that a great many different isolates of the HIV have been identified wherein sequence divergence of the PND has been noted. This diversity implies that if PNDs producing isolate specific immune responses are to be effective anti-HIV immunogens, then a mixture of PND peptides from different isolates may be the only solution to attainment of a broadly therapeutic or protective immunogen. In order to better characterize effective PND secondary structures, stable cyclics which avoid the use of labile disulfide bonding are needed. The cyclic peptides and the process for making these products disclosed in the instant invention satisfy this need.

Linear synthetic peptides have been prepared by a number of strategies conducted either in solution or on solid supports. Excellent texts covering the principles and techniques involved are: Principles of Peptide Synthesis, Bodanszky. M., Springer-Verlag (1984); Solid Phase Peptide Synthesis, Stewart J. M., Young, J. D., Pierce Chemical Company (2nd. ed. 1984); The Peptides, Gross, E., Meienhofer, J., Academic Press, Inc., (1979). In general, where cyclic peptides are required, disulfide bonded cyclics have been prepared by oxidation of linear synthetic peptides containing at least two sulfhydryls, for example two cysteines. The instant invention overcomes the problems associated with the use of labile disulfides by providing peptides having stable, amide-bonded cyclic structures.

Thus, this invention discloses novel HIV PND peptides having stable cyclic structures, a process for making, and a method of using such compounds. The stable cyclic HIV PND peptides, cPNDs, are prepared by amide bond formation between a free amino group on the amino terminal side of the loop amino acids and a free carboxyl group on the carboxy-terminal side of the loop amino acids, preferably the free carboxy terminus of the peptide. The cPNDs so formed are useful reagents for preparing covalent conjugate immunogens containing said cPNDs. Such conjugates are useful for raising mammalian anti-peptide, anti-HIV, or HIV-neutralizing immune responses. Compositions containing such conjugates may be used as a vaccine to prevent HIV-Disease, including Acquired Immune Deficiency Syndrome, (AIDS), or AIDS related complex (ARC) or as an immunogen for treating humans afflicted with AIDS or ARC. In addition, such conjugates provide a useful laboratory tool for analyzing the structure-function relationship involved in peptide elicitation of mammalian HIV-neutralizing immune responses.

## SUMMARY OF THE INVENTION

This invention is concerned with novel, stable, cyclic HIV PND peptides, having the structure:

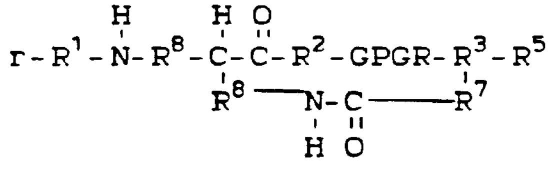

or pharmaceutically acceptable salts thereof, wherein:

r is

a) hydrogen,

b) benzyloxycarbonyl,
c) Ac-Cys, or
d) Ac-Cys(Acm);

$R^1$ is:
a) a bond,
b) a peptide of 1 to 5 amino acids optionally including a marker amino acid, such as norleucine, ornithine, β-alanine, and gamma amino butyric acid;

$R^2$ is :
a) a bond or a peptide of up to 17 amino acids, if $R^3$ is a peptide of at least 2 amino acids, or
b) a peptide of between 2 to 17 amino acids, if $R^3$ is a bond;

$R^3$ is:
a) a bond to $R^7$ or a peptide of up to 17 amino acids, if $R^2$ is a peptide of at least 2 amino acids, or
b) a peptide of between 2 to 17 amino acids, if $R^2$ is a bond;

-GPGR- is the tetramer -GlyProGlyArg-;

$R^5$ is bonded to a loop amino acid if $R^3$ is a bond, or to $R^3$ if $R^3$ is an amino acid or a peptide, and is selected from:
a) a peptide of one to five amino acids,
b) a marker amino acid,
c) -OH,
d) -COOH,
e) $-CONH_2$, or
f) absent;

$R^7$ is:
a) a bond from $R^3$ to the carbonyl carbon of the cycle;
b) lower alkyl,
c) substituted lower alkyl,
d) lower heteroalkyl,
e) substituted lower heteroalkyl, or
f) $-CH_2CH_2CH(CONH_2)NH-$;

$R^8$ is a bond or a lower alkyl of between one and 8 carbons.

Peptides having this structure are prepared by cyclizing linear HIV PND peptides, which are made by known solid-phase chemical synthetic methods incorporating appropriate side-chain protection. Following synthesis, the linear HIV PND peptide is cleaved from the resin, and cyclized in solution by treatment with a reagent capable of mediating amide bond formation, preferably diphenylphosphorylazide (DPPA), benzotriazol-1-yloxytris(dimethylamino)phosphonium hexafluorophosphate (BOP), or similar reagent A free amino group on one side of the loop amino acids and a free carboxyl group on the other side of the loop amino acids participate in formation of the cyclic structure. The amino group may be provided by the free amino terminus of the peptide, preferably by a norleucine which allows for easy peptide quantitation, by the free amino group of an amino-terminal isoglutamine (iQ), or by the ε-amino or α-amino group of a lysine on the amino-terminal side of the loop amino acids. The carboxyl may be provided by either the free peptide carboxy terminus or by the side chain of an acidic amino acid such as glutamate or aspartate.

The stable, amide-bonded cyclic peptides are useful as analytical tools, as reagents in ELISA assays, or as reagents for conjugation to an immunogenic carrier which may be comprised of polysaccharide, protein, both polysaccharide and protein, or any other material which confers enhanced immunogenicity on the cyclic peptides. Such conjugates are useful for inducing mammalian anti-peptide, anti-HIV, or HIV-neutralizing immune responses and for formulating vaccines to prevent HIV-Disease, including AIDS or ARC, or for treating humans afflicted with HIV-Diseases, such as AIDS of ARC.

## OBJECTS OF THE INVENTION

Accordingly, it is an object of this invention to provide novel, stable, and cyclic HIV PND peptides which are useful as reagents for preparing conjugates which may be used to elicit mammlian anti-peptide, anti-HIV, or HIV-neutralizing immune responses, or to prepare compositions for use as anti-HIV vaccines or as immunogens for treatment of humans afflicted with HIV-Diseases, including AIDS and ARC. Another object is to provide a process for the stable cyclization of HIV PND peptides through amide bonded structures.

DEFINITIONS AND ABBREVIATIONS

| | |
|---|---|
| AA assay | amino acid analysis method wherein peptides or proteins are acid hydrolyzed to the free amino acids and then quantitated |
| Acm | acetamidomethyl; thiol protecting group |
| activation | reaction of peptides, proteins, or polysaccharide moieties with a reagent capable of derivatizing the moiety in order to enable subsequent desirable reactions to occur |
| AIDS | Acquired Immune Deficiency Syndrome |
| amino acid | a molecule having both an acid and amino functional group; there are 20 common α-amino acids with the general structure $H_2N$-CHR-COOH, wherein the R group defines the identity of the amino acid; these amino acids may have either a D or L stereochemical form and unless specified otherwise, by the lower case one letter abbreviation, or by the prefix "D-" before an amino acid name, the amino acid is of the natural or L configuration; the names of the 20 common amino acids and the structure of the R group are identified herein in single-letter code according to the following table: |

| AMINO ACID NAME | 3-letter code | 1-letter code | side-chain (R) |
|---|---|---|---|
| Alanine | Ala | A | $-CH_3$ |
| Arginine | Arg | R | $-(CH_2)_3NHCHNH_2NH_2^+$ |
| Asparagine | Asn | N | $-CH_2CONH_2$ |
| Aspartic Acid | Asp | D | $-CH_2COOH$ |
| Cysteine | Cys | C | $-CH_2SH$ |
| Glutamic Acid | Glu | E | $-(CH_2)_2COOH$ |
| Glutamine | Gln | Q | $-(CH_2)_2CONH_2$ |
| Glycine | Gly | G | -H |
| Histidine | His | H | $-CH_2$-imidazole |
| Isoleucine | Ile | I | $-CH(CH_3)CH_2CH_3$ |
| Leucine | Leu | L | $-CH_2CH(CH_3)_2$ |
| Lysine | Lys | K | $-(CH_2)_4NH_3^+$ |
| Methionine | Met | M | $-(CH_2)_2SCH_3$ |
| Phenylalanine | Phe | F | $-CH_2$-Phenyl |
| Proline | Pro | P | -α,N-trimethylene |
| Serine | Ser | S | $-CH_2OH$ |
| Threonine | Thr | T | $-CH(OH)CH_3$ |
| Tryptophan | Trp | W | $-CH_2$-indole |
| Tyrosine | Tyr | Y | $-CH_2$-phenyl-OH |
| Valine | Val | V | $-CH(CH_3)_2$ |

| | |
|---|---|
| antibody | a protein produced by mamalian B cells that is capable of binding a particular antigen |
| ARC | AIDS-Related Complex |
| AZT | Azidothymidine, an anti-AIDS compound |
| bigeneric spacer | a molecular chain or conjugate resulting from the reaction of separately derivatized partners; analytical degradation of the conjugate formed through this spacer allows release and quantitation of the spacer, providing a meas- |

| | |
|---|---|
| | ure of the degree of covalent attachment |
| BOP | benzotriazol-1-yloxytris(dimethylamino)phosphonium hexafluorophosphate |
| capping | the elimination of reactive sites on a conjugate by reaction with small molecules |
| Cbz | benzyloxycarbonyl |
| conjugate | a complex of discrete chemical entities covalently bound one to the other, wherein at least one entity is a desired antigen (e.g. an HIV PND) and another entity is a carrier |
| Core amino acids | those amino acids of an HIV PND which are essential for inducing HIV-neutralizing immune responses in a mammal |
| DPPA | diphenylphosphorylazide |
| ELISA | enzyme-linked immunosorbant assay |
| Fmoc | 9-fluorenylmethyloxycarbonyl |
| HIV | Human Immunodeficiency Virus, a member of the lentivirus group and the purported etiologic agent implicated in AIDS and related complexes; HIV is alternatively known as HTLV (Human T-cell Lymphocyto-trophic Virus), LAV (Lymphadenopathy Associated Virus), and ARV (AIDS Related Virus) |
| immunogen | a molecule useful as a stimulator of a mammalian immune response |
| immunologically equivalent peptides | cyclic or linear peptides having in common the function of eliciting HIV neutralizing immune responses in mammals, such as antibodies which are able to recognize the equivalent peptide epitopes |
| marker amino acid | an amino acid having a signal in the AA assay which is free of interference by signals generated by other peptide or protein amino acids, for example, norleucine, ornithine, β-alanine, gamma amino butyric acid |
| Mtr | 4-methoxy-2,3,6-trimethylphenyl sulfonyl |
| NEM | N-ethylmaleimide |
| OMPC | Outer Membrane Protein Complex of Neisseria meningitidis; used as an immunoenhancer and peptide carrier |
| peptide | a polymer of amino acids linked by amide (peptide) bonds |
| PEP | peptide |
| PND | Principal neutralizing Determinant; the name attributed to peptidyl sequences capable of binding to HIV neutralizing antibodies and capable of raising HIV-neutralizing antibodies in a mammalian recipient upon inoculation with an immunogen containing the PND; for example, residues 296-341, or subfragments thereof, of HIV gp120 |
| PnPs6B | Streptococcus pneumoniae 6B capsular polysaccharide |
| PRO | an immunogenic protein |
| protein | a large peptide |
| PRP | Polyribosyl-ribitol phosphate |
| PSA | anionic polysaccharide, usually having repeat phosphate units in the monomer unit of the polymer |
| resins | solid support matrices for solid phase peptide synthesis<br>Wang:<br>4-(hydroxymethyl)phenoxymethyl linkage to copolystyrene-1%divinylbenzene resin, which is used for batch Fmoc solid phase peptide synthesis, with final 95% TFA cleavage from the resin and concomitant deprotection of acid sensitive side chain protecting groups;<br>Sasrin:<br>4-(hydroxymethyl)-3-methoxyphenoxymethyl linkage to copolystyrene-1%divinylbenzene resin, which is used for batch Fmoc solid phase peptide synthesis, with final 1% TFA/$CH_2Cl_2$ cleavage from the resin, leaving intact acid labile side chain protecting groups;<br>Pepsyn KA:<br>4-(hydroxymethyl)phenoxymethyl linkage to polyamide resin adsorbed on to kieselguhr, which is used for continuous flow column Fmoc solid phase peptide synthesis. Peptides are cleaved from the resin as described above for Wang resin; |

| | |
|---|---|
| | Pepsyn KH: 4-(hydroxymethyl)-3-methoxymethyl linkage to polyamide resin adsorbed on to kieselguhr, which is used for Fmoc solid phase peptide synthesis. Side chain protected peptides are cleaved from the resin as described above for the Sasrin resin |
| "scaffold" | immunogen having multiple peptide epitopes built upon a carrier molecule |
| SCMHC | S-carboxymethyl homocysteamine, an acid-stable bigeneric spacer released by degradation of covalent conjugate immunogens and quantifiable by AA assay |
| SCMC | S-carboxymethyl cysteamine, an acid-stable bigeneric spacer released by degradation of covalent conjugate immunogens and quantifiable by AA assay |
| Z | benzyloxycarbonyl |

## DETAILED DESCRIPTION OF THE INVENTION

This invention is concerned with novel, cyclic HIV PND peptides having the structure cPND

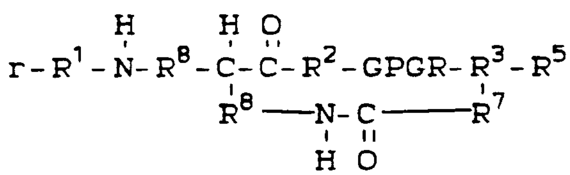

or pharmaceutically acceptable salts thereof, wherein:

r is:
a) hydrogen,
b) benzyloxycarbonyl,
c) Ac-Cys, or
d) Ac-Cys(Acm);

$R^1$ is:
a) a bond,
b) a peptide of 1 to 5 amino acids, optionally including a marker amino acid, such as norleucine, ornithine, β-alanine, and gamma amino butyric acid;

$R^2$ is :
a) a bond or a peptide of up to 17 amino acids, if $R^3$ is a peptide of at least 2 amino acids, or
b) a peptide of between 2 to 17 amino acids, if $R^3$ is a bond;

$R^3$ is
a) a bond to $R^7$ or a peptide of up to 17 amino acids, if $R^2$ is a peptide of at least 2 amino acids, or
b) a peptide of between 2 to 17 amino acids, if $R^2$ is a bond;

-GPGR-is the tetramer -GlyProGlyArg-;

$R^5$ is bonded to a loop amino acid if $R^3$ is a bond, or to $R^3$ if $R^3$ is an amino acid or a peptide, and is selected from:
a) a peptide of one to five amino acids,
b) a marker amino acid,
c) -OH,
d) -COOH,
e) $-CONH_2$, or
f) absent;

$R^7$ is:
a) a bond from $R^3$ to the carbonyl carbon of the cycle,
b) lower alkyl,
c) substituted lower alkyl,
d) lower heteroalkyl,
e) substituted lower heteroalkyl, or
f) $-CH_2CH_2CH(CONH_2)NH-$; and

$R^8$ is a bond or a lower alkyl of between one and 8 carbons.

Lower alkyl consists of straight or branched chain alkyls having from one to eight carbons; substituted lower alkyl may be mono- or di-substituted with $-NH_2$, $-CONH_2$; heteroalkyl may contain one or two heteroatoms selected from oxygen, nitrogen, or sulfur.

Hereinafter, amino acids $-R^2$-GPGR-$R^3$-, which go toward formation of the loop of a cyclic peptide, will be referred to as loop amino acids. The term "nonlabile bond" means a covalent linkage, other than a labile disulfide bond, such as amide and thioether bonds. By using an appropriate bridge structure, the conformation of the loop structure of the cycle may be optimized allowing the fine tuning of the PND epitope to be presented to the immune system. For example, use of a bridge structure containing 2 carbons generates a "tighter" loop than when a $C_5$-containing bridge is used.

Accordingly, in a preferred embodiment of the invention, a cyclic peptide having the structure:

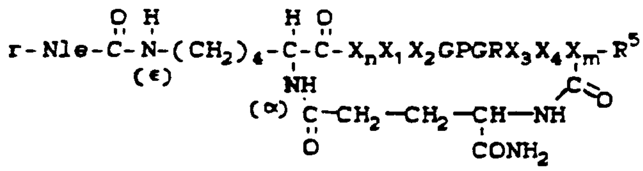

is prepared by cyclizing a linear peptide having the structure:

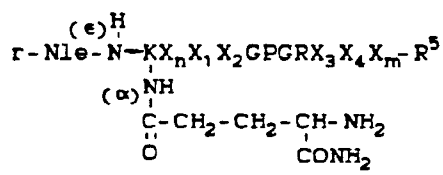

wherein:

the squiggly line represents $-(CH_2)_4-$;

-GPGR- is the tetramer -GlyProGlyArg-;

$X_1$ is a constituent of $R^2$ selected from:

a) serine,
b) proline,
c) arginine,
d) histidine,
e) glutamine, or
f) threonine;

$X_2$ is a constituent of $R^2$ selected from:

a) isoleucine,
b) arginine,
c) valine, or
d) methionine;

$X_n$ is is a constituent of $R^2$ and is either a bond or a peptide of up to 15 amino acids;

$X_3$ is a constituent of $R^3$ selected from:

a) alanine,
b) arginine, or
c) valine;

$X_4$ is a constituent of $R^3$ and is selected from:

a) phenylalanine,
b) isoleucine,
c) valine, or
d) leucine; and

$X_m$ is a constituent of $R^3$ and is a bond or a peptide of up to 15 amino acids.

In another preferred embodiment of the invention, the cyclic peptide having the structure:

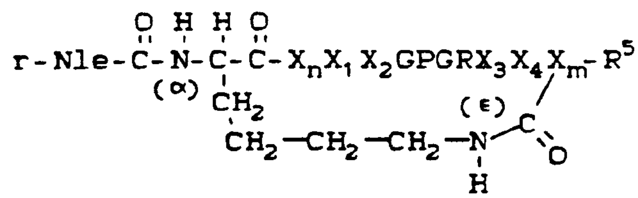

is prepared by cyclizing a linear peptide having the structure:

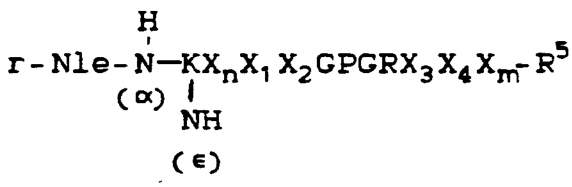

wherein all variables are as defined above.

The novel cyclic peptides of this invention are prepared in essentially two phases: First, the linear peptide is prepared, for example on an ABI-431A peptide synthesizer, by known solid phase peptide synthetic chemistry, for example using Fmoc chemistry and appropriately side-chain protected Fmoc-amino acids as reagents.

Second, the linear peptide is cleaved from the resin and cyclized in solution by allowing the free amino terminus of the peptide, the free amino group of an amino terminal isoglutamine, or a free ε-amino or α-amino group of a lysine on one side of the loop amino acids to be amide bonded to a free carboxyl group on the carboxy-terminal side of the loop amino acids through DPPA, BOP, or similar reagent mediated peptide bond formation.

Products obtained may be characterized by fast atom bombardment-mass spectrometry [FAB-MS], reverse phase HPLC, amino acid analysis, or nuclear magnetic resonance spectroscopy (NMR).

Examples 1-25 show the synthesis of species of the invention.

The cyclic peptides of this invention may be used as reagents in ELISA assays, and provide a tool for analyzing peptide structure function relationships when HIV-neutralizing antibodies are used in the assay. Alternatively, the cyclic peptides of the invention may be conjugated to a carrier, for example a polysaccharide, an immunogenic protein, or a combination of any material capable of enhancing the immunogenicity of the peptide. For example, peptides of this invention may be conjugated to a carrier comprising polyribosyl ribitol phosphate (PRP) from the capsular polysaccharide of Haemophilus influenzae b, and the outer membrane protein complex (OMPC) of Neisseria meningitidis b. Examples are provided below wherein such conjugates are prepared.

The following examples are provided to more particularly demonstrate how to make and use the cyclic peptides of this invention. However, the examples provided are not to be construed so as to limit the scope of the invention.

A. Examples of Peptides prepared according to this Invention:

EXAMPLE 1

Solution Synthesis of Peptide Bonded cPND7:

The linear peptide Cbz-Nle-Lys(Boc)-His(Trt)-Ile-Gly-Pro-Gly-Arg(Mtr)-Ala -Phe was synthesized following solid-phase methods on an ABI 431A peptide synthesizer using 373 milligrams (01 mmoles) of commercially available Fmoc-Phenylalanyl-p- alkoxybenzyl alcohol resin. With the exception of norleucine, which was purchased in the benzyloxycarbonyl (Cbz) protected form, L-amino acids used were the fluorenylmethoxycarbonyl (Fmoc) derivatives having the appropriate acid-labile side chain protecting groups. The polypeptide-derivatized resin product was transferred to a sintered glass funnel, washed with dichloromethane, and dried, to yield 0.6 g of polypeptide-resin-product.

The peptide was cleaved from the resin by treatment with 6 ml of a 95:2:3 mixture of TFA:1,2 ethanediol:anisole for 16 hours. The reaction mixture was filtered through a sintered glass funnel, the resin washed with 10 ml TFA, and the filtrates combined. Following concentration to about 1 to 2 ml of yellow oil, the linear peptide was recovered by trituration with 400 ml of diethyl ether, in 50 ml portions, and filtration on a sintered glass funnel. Dissolution with 100 ml 1% TFA followed by lyophilization yielded 298 mg of linear peptide.

The peptide powder was dissolved in 800 ml DMF, neutralized with 0.42 ml diisopropylethylamine, and treated with 0.077 ml diphenylphosphorylazide. The solution was stirred in the dark for 70 hours at 4°C to allow formation of the cyclic lactam. After quenching by addition of 3 ml glacial acetic acid, the reaction mixture was concentrated to about 1 to 2 ml of oil, dissolved in 10% aqueous acetic acid, and lyophilized.

The cyclic peptide was purified by G-15 size exclusion chromatography using 5% acetic acid as the mobile phase. Fractions, monitored by UV detection, containing the peptide were pooled and lyophilized to yield 135 mg of dry cyclic peptide. All results obtained were consistent with the structure cPND7:

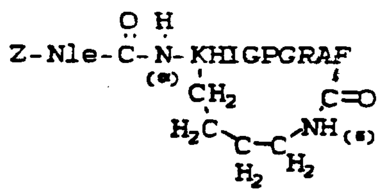

which may also be represented as:

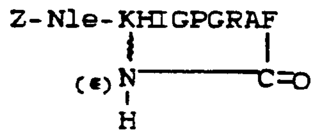

EXAMPLE 2

Deprotection of cPND7 to yield the hydrogen form, cPND8:

Deprotection of cPND7 was achieved by dissolving the cyclic peptide in 20 ml of 30% aqueous acetic acid and hydrogenation at 40 psi for 16 hours over 100 mg of 10% palladium on carbon. The reaction mixture was filtered over celite to remove the catalyst, and the filtrate was lyophilized. Reverse phase HPLC using a Vydac C18 semi-prep column was utilized to obtain 8.5 mg of pure deprotected cyclic peptide. This method of deprotection is applicable to all peptides synthesized as the benzyloxycarbonyl N-protected peptide, to yield the free hydrogen form of the peptide. The structure of the product was confirmed by FAB-MS, analytical HPLC and amino acid analysis, and all results were consistent with the structure cPND8:

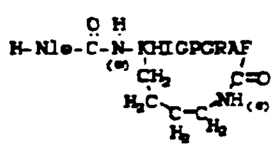

which may also be represented as:

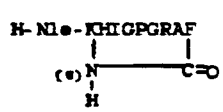

EXAMPLE 3

Synthesis of cPND10:

The synthesis of cPND10, having an Acm protected Ac-cysteine at the peptide amino-terminus, is identical to the procedure used in Example 1, except that the synthesis here included an Fmoc-norleucine, rather than the Z-Nle, and the additional amino acid, Ac-Cys(Acm) was used as the N-terminal amino acid. Thus, the linear peptide Ac-Cys(Acm)-Nle-Lys(Boc)-His(Trt)-Ile-Gly-Pro-Gly-Arg(Mtr)-Ala-Phe, was assembled using commercially available Fmoc-Nle, and Fmoc-Cys(Acm). This modification of the Example 1 procedure is applicable to the synthesis of other cPND peptides where an N-terminal Ac-Cys(Acm) is desirable.

EXAMPLE 4

Deprotection of cPND10 to yield cPND9:

The Acm protected Ac-Cys(Acm) may be converted to the free Ac-Cys-SH (free sulfhydryl) form of the peptide according to the procedure described in Atherton, E. et al., Chem. Soc. Perkin Trans., I, 2057 (1985). This procedure is applicable to removal of Acm thiol protection of peptides in preparation for conjugation with a thiophilic agent, such as bromoacetylated or maleimidated proteins or polysaccharides. A portion of cPND10 was dissolved in 10% aqueous acetic acid and treated with mercuric trifluoroacetate (10-fold excess). The pH was readjusted to 4 and the solution was stirred at room temperature while cleavage of the S-Acm groups was monitored by reverse-phase HPLC. When the reaction was judged complete, the solution was saturated with hydrogen sulfide gas. The mercury(II) sulfide precipitate was removed by centrufugation, and cPND9 was purified by RP-HPLC. The structure and purity of cPND9 was confirmed by FAB-MS, analytical HPLC, and amino acid analysis.

EXAMPLES 2-21

cPND Peptides Synthesized According to the Methods of Examples 1-4 and 20-21:

The procedures established above in Examples 1-4 and below in Examples 20-21 for the synthesis of cPND7, cPND8, cPND9, cPND10, cPND31, and cPND32 may be generally applied, without any substantial modification, aside from changes in the peptide primary sequence and inclusion of appropriate protecting groups, in the synthesis of the cyclic form of synthetic PND peptides from many different isolates. Thus, all of the following peptides were synthesized according to these methods:

| EX. # | NAME | STRUCTURE | FAB MS M+H |
|---|---|---|---|
| 2) | cPND8 | 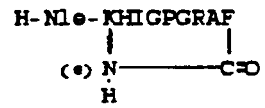 , | 1077 |
| 3) | cPND10 | 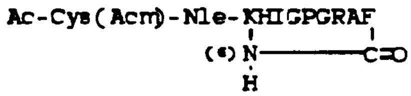 , | 1293 |
| 4) | cPND9 | 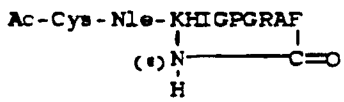 , | 1223 |
| 5) | cPND11 | 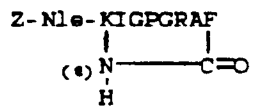 , | 1074 |
| 6) | cPND12 | 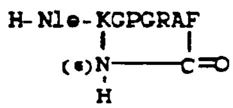 , | 827 |
| 7) | cPND13 | 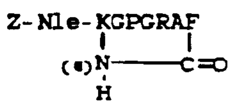 , | 961 |
| 8) | cPND14 | 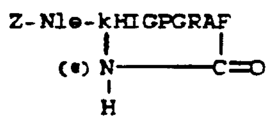 , | 1211 |
| 9) | cPND15 | 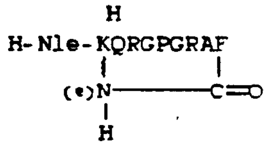 , | 1111 |

| EX. # | NAME | STRUCTURE | FAB-MS $M^+H$ |
|---|---|---|---|
| 10) | cPND16 | 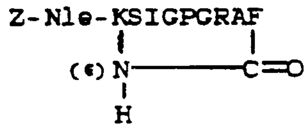 , | 1161 |
| 11) | cPND21 | 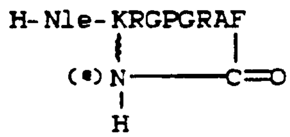 , | 983 |
| 12) | cPND23 | 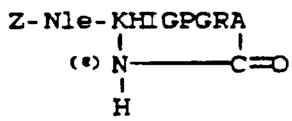 , | 1063 |
| 13) | cPND24 | 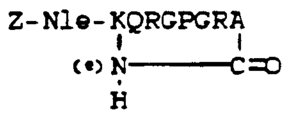 , | 1098 |
| 14) | cPND25 | 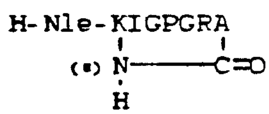 , | |

| Ex. # | Name | Structure | FAB-MS M+H |
|---|---|---|---|
| 15) | cPND26 | 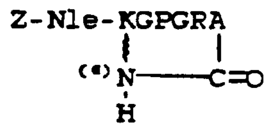 , | 814 |
| 16) | cPND27 | 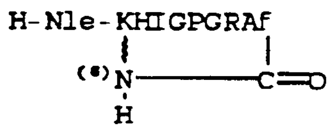 , | 1077 |
| 17) | cPND28 | 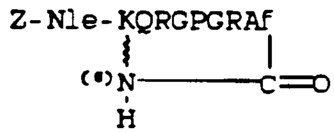 , | 1245 |
| 18) | cPND29 | 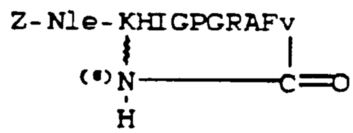 , | 1310 |
| 19) | cPND30 | 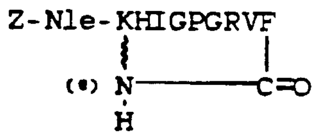 , | 1257 |
| 20) | cPND31 | 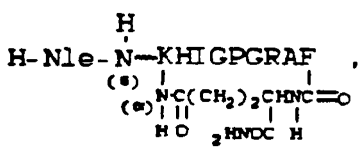 , | 1204 |
| 21) | cPND32 | 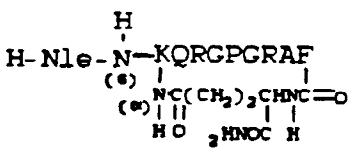 . | 1238 |

EXAMPLE 20

Synthesis of cPND31:

Two grams (0.6 meq/gram) of Fmoc-Phe-Wang resin was loaded on an ABI 431A synthesizer. Fmoc single coupling protocols were used to add Fmoc-Ala, Fmoc-Arg(Tos), Fmoc-Pro, Fmoc-Ile, Fmoc-His(Trt), Boc-Lys-(Fmoc), and Cbz-Nle to produce 3.7 grams of linear peptide resin having the sequence:
Boc-Lys($N^{\varepsilon}$-Z-Nle)-His(Trt)-Ile-Gly-Pro-Gly-Arg(Tos)-Ala-Phe.

The peptide was cleaved from the resin by treating with 95% TFA, 5% water for two hours. The resin was removed by filtration, the TFA removed from the filtrate by evaporation in vacuo, and the residue was triturated with diethyl ether. The precipitate was recovered by filtration and dried to yield 1.7 grams of linear peptide having the sequence:
H-Lys($N^{\varepsilon}$-Z-Nle)-His-Ile-Gly-Pro-Gly-Arg(Tos)-Ala-Phe.

The peptide was treated with Boc-isoglutamine-ONp (0.71 grams, 2 nmoles,) and DIEA (0.35 ml, 2 mmoles)

in DMF (10 ml) overnight at room temperature. The DMF was evaporated, and the residue treated with diethyl ether. The precipitate was recovered by filtration and washed with ethyl acetate. The dried peptide (1.9 grams) was treated with TFA (100 ml) for 0.5 hours. The TFA was evaporated in vacuo, the residue triturated with diethyl ether and the precipitate was recovered by filtration and dried.

The peptide was desalted on Sephadex G-10 in 10% aqueous acetic acid as the eluent. Peptide fractions were lyophilized to yield 1.2 grams (0.79 mmoles) of:

H-isoGln-Lys($N^ε$-Z-Nle)-His-Ile-Gly- Pro-Gly-Arg(Tos)-Ala-Phe

Two batches (0.55 gm, 0.36 mmoles) of the peptide were separately dissolved in 1000 mL ice cold DMF and DIEA (0.16 mL, 0.9 mmoles) and DPPA (0.12 mL were added and the solutions were stirred overnight at room temperature. The DMF was evaporated in vacuo and the residues combined and solubilized in $CHCl_3$. The organic fraction was washed with 57 aqueous citric acid, then dried over $MgSO_4$ and evaporated to yield 0.78 gm of crude cyclic peptide. This material was treated with liquid HF (10 mL) containing anisole (1 mL) for two hours at 0°C. The HF was evaporated and the residue was purified by gradient elution on reversed phase HPLC (Vydac C-18, 0-50% $CH_3CN$, over 50 minutes using 0.1 % aqueous TFA as the buffer) to give 250 mg of pure cPND31 (M+H=1204).

## EXAMPLE 21

Synthesis of cPND32:

Essentially the same procedure used in Example 20 for synthesis of cPND 31 was employed here except that the linear peptide that was cyclized had the sequence:

H-isoGln-Lys($N^ε$-Z-Nle)-Gln-Arg(Tos)-Gly-Pro-Gly-Arg(Tos)-Ala-Phe.

B. Examples of Conjugates prepared using Peptides of the Invention:

## EXAMPLE 22

Preparation of the N-(bromoacetyl)-6-aminocaproic derivative of OMPC, (BrAc-6-ACA-OMPC):

An OMPC solution (10 ml) (59 mg/ml) was centrifuged at 43K rpm, 4°c for 2 hours. The pellet was resuspended using a Dounce homogenizer in 6 ml of pH9 (Kolthoff) buffer and 1 milliliter of a N-(bromoacetyl)-6-aminocaproic acid p-nitrophenyl ester solution (85 mg/ml of acetonitrile) was added. The resultant mixture was agitated for 45 hours. Insoluble material was pelleted by a low speed centrifugation and the supernatant was then centrifuged at 43K rpm, 4°C for 2 hours. The pellet was resuspended on 10 ml of $H_2O$ and recentrifuged at 43K, 2 hours at 4°C. This pellet was resuspended in 10 ml of $H_2O$ affording the BrAc-6-ACA-OMPC. The amino acid analysis showed 268 nanomole/ml of -6-aminocaproic acid and 196 nanomoles/ml of lysine. The protein concentration was 2.4 mg/ml (41% recovery). To assay bromoacetylating potential, 1 ml of this solution was incubated at pH 8 with 30 μl of N-acetylcysteamine. After dialysis to remove excess reagent, the solvent was evaporated to yield the title compound. An aliqout of the sample was acid hydrolyzed and assayed by Spinco, revealing 54 nanomoles/ml S-carboxymethyl cysteamine and 140 namomoles/ml lysine (SCMC/lys = 0.38). This indicates that 38% of the lysines present are bromoacetylating moieties.

## EXAMPLE 23

Preparation of the cystamine (Bis-2-aminoethyl disulfide) derivative of PRP. (PRP-cys-$NH_2$):

The tetrabutyl ammonium salt of PRP (300 mg) was dissolved in 11 ml of dimethyl formamide (DMF). Subsequently, 30 mg of carbonyldiimidazole was added and the solution stirred at room temperature (r.t.) for 1 hour. The DMF solution was then added with stirring to a chilled (ice) solution of cystamine dihydrochloride (450 mg/10ml $H_2O$; pH adjusted to 10.3 with NaOH) and stirring continued for 15 min. in the ice bath. The solution was then removed from the ice bath and aged at room temperature for 45 min. after transfer to a dialysis bag. Dialysis against (vs.) successive buffer changes was then effected as follows:

(a) vs. 4L pH 7, 0.1M $PO_4$ for 4.2 hours
(b) vs. 4L pH 7, 0.01M $PO_4$ for 8 hours;
(c) vs. 4L pH 7, 0.01M $PO_4$ for 16 hours and
(d) vs. 16L $H_2O$ for 8 hours.

Lyophilization afforded 150 mg of the title compound, cystamine derivative of PRP. The NMR of this material confirmed the cystamine derivatization of PRP. By comparing the two methylene resonances centered at 3 ppm ($CH_2$-S) with the glycosidic proton (5.1 ppm) or the total PRP proton integral, an average value of 48 cystamine moieties per 100 ribosyl-ribitol phosphate moieties (i.e. PRP monomeric unit) was calculated.

EXAMPLE 24

Reduction of $PRP\text{-}cys\text{-}NH_2$ to Yield PRP-SH:

To 9.0 ml of a pH 8.0 buffer (0.01M in $PO_4$, 0.005M in EDTA, = buffer A) was added 40 mg of $PRP\text{-}cys\text{-}NH_2$ (see Example 23 for preparation). After complete dissolution (15 min), 42 mg of solid dithiothreitol (DTT) was added. The mixture was degassed, nitrogenated, and aged for 4.5 hours at room temperature. The solution was then transferred to dialysis tubing and dialyzed against the buffer changes as follows:

1) vs. 4L buffer A for 16 hours;

2) vs. 4L buffer A for 7.25 hours;

3) vs. 1L of a pH 8, 0.1M $PO_4$ buffer, 0.005M in EDTA (buffer B) for 17 hours.

At this point an Ellman assay of the solution indicated a thiol titer of 2.04 mmoles SH/ml and therefore a total of 15.3 mmoles of the title PRP-SH for a volume of 7.5 ml.

EXAMPLE 25

Preparation of cPND9-PnPs6B-OMPC (cPND9 =

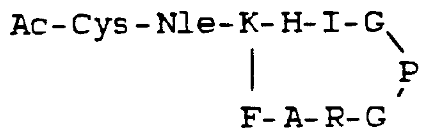

):

PnPs6B(n-Bu4N+):

PnPs6B (484 mg) was dissolved in distilled water (48 mL) and the solution magnetically stirred until all solids went into solution (1.5 h). While the polysaccharide was dissolving, a column (20 mm) was charged with Dowex 50 X 2 ($n\text{-}Bu_4N^+$) ion exchange resin (30 mL) and the resin bed washed with water for 1.5 hr. The viscous polysaccharide solution was applied to the rinsed resin and allowed to pass through the bed by gravity (16 h). The column was washed with water (8 mL) and the combined eluants lyophilized, providing 800 mg of a pale yellow solid that was dried in a vacuum desiccator (50 mm Hg, 60 h) over $P_2O_5$. This procedure provided 564 mg of dry PnPs6B tetra-n-butyl ammonium salt, PnPs6B(n-Bu4N+).

$PnPs6B\text{-}BuA_2$:

PnPs6B($n\text{-}Bu_4N^+$)(140 mg) was dissolved in dimethylsulfoxide (5 mL) and magnetically stirred for 15 min, at which time all solids appeared to be in solution. To this mixture was added 1,1′-carbonyldiimidazole (15 mg), in one portion, and the reaction stirred at room temperature (80 min). In a separate flask, a solution of butanediamine dihydrochloride ($BuA_2{\cdot}2HCl$, 344 mg) in water (5 mL) was made basic (pH 10.2) by the addition of 2.5 N NaOH then cooled to 0°C. To this cold mixture was added the activated polysaccharide, in a slow steady stream, and the resulting solution stirred at 0°C (30 min). The reaction mixture was allowed to warm up to room temperature and stirred for an additional 1 h, after which it was transferred to dialysis tubing (2 mL/cm) and dialyzed (4°C) against the following: 4 L 0.1 M pH 7.0 $HPO_4$ buffer, 5 h; 4 L 0.01 M pH 7.0 $HPO_4$ buffer, 15 h; 4 L 0.01 M pH 7.0 $HPO_4$ buffer, 9 h; and 4 L distilled $H_2O$, 15 h. The contents of the dialysis tubing were lyophilized, providing a white solid that was dried overnight (16 h) in a desiccator over $P_2O_5$, to yield 94 mg of PnPs6B-Butane diamine ($PnPs6B\text{-}BuN_2$). The NMR (300 MHz, $D_2O$) of this material revealed that a 50% loading (50 $BuA_2$ units per 100 6B monomer units) of butanediamine was achieved. The percent loading was determined by comparing the integration of the butanediamine methylene protons to rhamnose methyl protons.

PnPs6B-BuA2-BrAc:

$6B\text{-}BuA_2$ (94 mg) was dissolved in pH 9.04 Kolthoff buffer (10 mL) and the mixture magnetically stirred for 30 min to effect solution. To this aqueous solution was added a mixture consisting of p-nitrophenyl bromoacetate (94 mg) in acetonitrile (1 mL) and the reation stirred overnight (22 h) in the cold-room (4° C). The resulting viscous yellow solution was transferred to dialysis tubing (2 mL/cm) and dialyzed (4°C) against the following: 15 L distilled $H_2O$, 23 h; and 4L distilled $H_2O$, 23 h. The contents of the dialysis bag were stored as a solution. One mL of this solution was lyophilized, providing 3.5 mg of $PnPs6B\text{-}BuA_2\text{-}BrAc$ as a white solid. The NMR (300 MHz, $D_2O$) of this material showed that the loading of butanediamine was 28°%. The discrepancy between

this value and the one obtained earlier (on PnPs6B-$BuA_2$) may be due to a solubility problem since not all of the lyophilized material would go back into solution.

PnPs6B-cPND9:

Aqueous PnPs6B-$BuA_2$-BrAc (11 mL, approx. 38.5 mg) was added to a vial containing the cyclic , peptide

cPND9 (cPND9 = 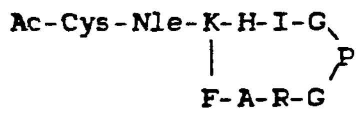 )

(8.5 mg, 6.9 μmol, molecular weight of 1221). An Ellman's test was immediately performed on the resulting solution (200 μL sample) and showed an SH titer ($OD_{412}$=0.270) of 3.3 μmole. The reaction mixture was transferred to a 15 mL centrifuge tube, degassed and blanketed with nitrogen then tumbled at room temperature (5 h). The reaction mixture was transferred to dialysis tubing (2 mL/cm) and dialyzed against 4L distilled water (4°C, 23 h). One mL of the solution contained within the dialysis tubing was removed and lyophilized. The remaining (13.5 mL) solution's pH was adjusted to pH 83 by the addition of dried pH 8.0 $HPO_4$ buffer salt (180 mg) and a few drops of 5 N NaOH. This pH-adjusted solution was used for conjugation with thiolated OMPC.

cPND9-PnPs6B-OMPC:

Sterile OMPC (10 mL, approx. 45 mg) was pelleted down by ultracentrifugation (4°C to 20°C, 43 K rpm, 1.5 h). The pellet was resuspended in 6 mL of a sterile-filtered (Millipore Millex-GV 0.22 μm sterile filter) thiolation mixture which consisted of the following: homocysteinethiollactone hydrochloride (86 mg), ethylenediaminetetraacetic acid disodium salt (86 mg), and dithiothreitol (17 mg) in pH 11 borate buffer (10 mL). The pellet was homogenized (dounce) and transferred to a sterile 15 mL centrifuge tube, degassed and blanketed with nitrogen. The reaction mixture was tumbled overnight (20.5 h) at room temperature, then transferred to an ultracentrifuge tube and topped with 1 M $KH_2PO_4$. The protein was pelleted down (4°C, 43 K rpm, 2 h), resuspended in 0.1 M $HPO_4$ buffer (10 mL), homogenized (Dounce) and repelleted (4°C, 43 K rpm, 2 h). The sterile protein pellet was used directly in the conjugation with the peptide-polysaccharide solution.

The sterile protein pellet was resuspended in sterile-filtered (Millipore Millex-GV 0.22 μm sterile filter) polysaccharide-peptide solution and homogenized (Dounce). An Ellman's test was performed immediately, and showed an SH titer of 15.3 μmol ($OD_{412}$=0.730). The reaction mixture was transferred to a sterile 15 mL centrifuge tube, degassed, blanketed with nitrogen, and aged overnight (21 h). An Ellman's test showed an SH titre of 8.2 μmol ($OD_{412}$=0.390). The protein was capped by the addition of 1 mL of a sterile-filtered (Millipore Millex-GV 0.22 μm sterile filter) solution consisting of the following: N-ethylmaleimide (75 mg) in pH 8.0 $HPO_4$ buffer (5 mL). This mixture was aged overnight (16 h) at room temperature. An Ellman's test was now visibly negative ($OD_{412}$=0.066, SH titer = 1.38 μmol). The sterile capped conjugate was pelleted by ultracentrifugation (4°C, 43 K rpm, 2 h), then resuspended and homogenized (Dounce) in sterile $H_2O$ (10 mL), and stored in a sterile plastic 15 mL centrifuge tube. The following assays were performed (amount solution used): Lowry protein, 1.50 mg/mL (100 μL); Spinco amino acid analysis, Nle= 2 nmoles/100 μl (100 μL). The peptide loading may be directly calculated from these values as the Nle concentration is the same as the peptide concentration using a conversion of 1221 for the peptide molecular weight. Thus, the peptide concentration was calculated to be 0.0242 mg/ml, yielding an peptide loading of 0.0161 mg PEP/mg OMPC for the title conjugate.

Example 26

Preparation of the cPND9-PRP-OMPC (cPND9 =

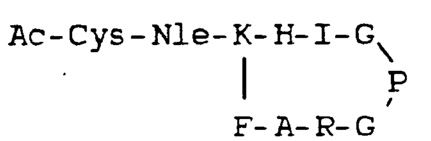

):

33 mg of PRP-$BuA_2$-BrAc (17 $BuA_2$-BrAc/100 PRP monomer units), prepared in the same manner as was

the PnPs6B-$BuA_2$-BrAc in Example 25, was dissolved in 3.5 mL pH8 (0.1M $PO_4$) buffer. The solution was degassed and the air exchanged for $N_2$, and 6.9 mg of cPND9 added. If pure, this corresponds with 5.65 mmoles of cyclic peptide (M.W 1221). However, an Ellman assay indicated the presence of only 1.5 μmoles of SH. The peptide-PRP solution was aged for 7 hr (Ellman=0) and then dialyzed vs. 4L of $H_2O$ for 16 hr. Freeze drying an aliquot afforded an NMR spectrum having phenyl resonances (7.28 and 7.43 ppm) diagnostic for the presence of peptide conjugated to the PRP.

OMPC was functionalized as usual with N-acetyl homocysteine thiolactone, affording a thiolated OMPC (7.35 μmoles SH/45 mg OMPC starting OMPC = 0.163 μmole SH/mg). The thiolated protein was resuspended in 3 mL of the peptidylated PRP which was first buffered to pH 8 with a phosphate salt and then filtered through a 0.22 μ sterile millex GV filter. This OMPC-peptidylated PRP mixture was degassed and aged for 41 hrs. At this time, about 55°% of the thiol titer remained. A sterile filtered solution (0.6 mL) of N-ethylmaleimide (31 mg/3.5 mL) in pH 8 buffer was added and the reaction mixture aged for 2.5 hrs. It was then free of thiol. The solution was diluted to 10 mL with $H_2O$ and centrifuged at 43,000 rpm, for 2 hr at 4°C. The pellet was resuspended in 10 mL of $H_2O$ and recentrifuged as above. The pellet from the second centrifugation was resuspended in 7 mL of $H_2O$ and aged 60 hr at 4°C. A low speed (clinical spin) pelleted a small amount of precipitate. The supernatant was assayed by Spinco, revealing a SCMHC/lys = 0.055, and a norleuciue (Nle) concentration of 4.8 nanomole/ml, which translates into a peptide concentration of 0.0056 mg PEP/mL (using 1221 as the molecular weight for the peptide). Protein (Lowry) assay showed the presence of 0.810 mg/mL. Thus, the peptide loading = 0.0072 for the title conjugate.

While the foregoing specification teaches the principles of the present invention, with examples provided for the purpose of illustration, it will be understood that the practice of the invention encompasses all the usual variations, adaptations, modifications, or deletions as come within the scope of the following claims and its equivalents.

## Claims

1. A cyclic HIV PND peptide having the structure:

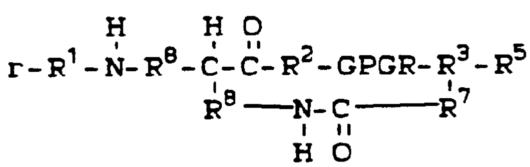

or pharmaceutically acceptable salts thereof, wherein:

r is
a) hydrogen,
b) benzyloxycarbonyl,
c) Ac-Cys, or
d) Ac-Cys(Acm);

$R^1$ is
a) a bond, or
b) a peptide of 1 to 5 amino acids, optionally including a marker amino acid;

$R^2$ is
a) a bond or a peptide of up to 17 amino acids, if $R^3$ is a peptide of at least 2 amino acids, or
b) a peptide of between 2 to 17 amino acids, if $R^3$ is a bond;

$R^3$ is:
a) a bond to $R^7$ or a peptide of up to 17 amino acids, if $R^2$ is a peptide of at least 2 amino acids, or
b) a peptide of between 2 to 17 amino acids, if $R^2$ is a bond;

-GPGR- is the tetramer -GlyProGlyArg-;

$R^5$ is bonded to a loop amino acid if $R^3$ is a bond, or to $R^3$ if $R^3$ is an amino acid or a peptide, and is selected from:
a) a peptide of one to five amino acids,
b) a marker amino acid,
c) -OH,
d) -COOH,
e) -$CONH_2$, or

f) absent;

$R^7$ is

a) a bond from $R^3$ to the carbonyl carbon of the cycle,
b) lower alkyl,
c) substituted lower alkyl,
d) lower heteroalkyl,
e) substituted lower heteroalkyl, or
f) $-CH_2CH_2CH(CONH_2)NH-$;

$R^8$ is a bond or a lower alkyl of between one and eight carbons.

**2.** The cyclic peptide of Claim 1 having the structure:

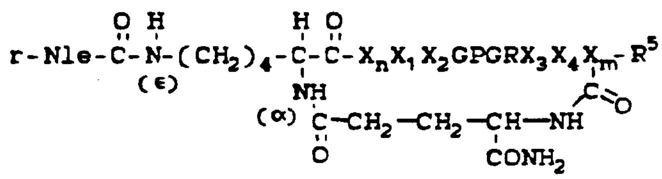

or pharmaceutically acceptable salts thereof, wherein:

-GPGR- is the tetramer -GlyProGlyArg-;

$X_1$ is a constituent of $R^2$ selected from:

a) serine,
b) proline,
c) arginine,
d) histidine,
e) glutamine, or
f) threonine;

$X_2$ is a constituent of $R^2$ selected from:

a) isoleucine,
b) arginine,
c) valine, or
d) methionine;

$X_n$is a constituent of $R^2$ and is either a bond or a peptide of up to 15 amino acids;

$X_3$ is a constituent of $R^3$ selected from:

a) alanine,
b) arginine, or
c) valine;

$X_4$ is a constituent of $R^3$ and is selected from:

a) phenylalanine,
b) isoleucine,
c) valine, or
d) leucine; and

$X_m$ is a constituent of $R^3$ and is a bond or a peptide of up to 15 amino acids.

**3.** The cyclic peptide of Claim 1 having the structure:

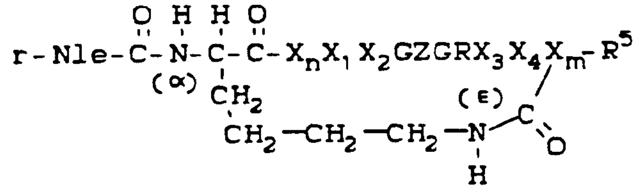

or pharmaceutically acceptable salts thereof, wherein:

-GPGR- is the tetramer -GlyProGlyArg-;

$X_1$ is a constituent of $R^2$ selected from:

a) serine,
b) proline,
c) arginine,
d) histidine,
e) glutamine, or
f) threonine;

$X_2$ is a constituent of $R^2$ selected from:

a) isoleucine,
b) arginine,
c) valine, or
d) methionine;

$X_n$ is is a constituent of $R^2$ and is either a bond or a peptide of up to 15 amino acids;

$X_3$ is a constituent of $R^3$ selected from:

a) alanine,
b) arginine, or
c) valine;

$X_4$ is a constituent of $R^3$ and is selected from

a) phenylalanine,
b) isoleucine,
c) valine, or
d) leucine; and

$X_m$ is a constituent of $R^3$ and is a bond or a peptide of up to 15 amino acids.

**4.** The peptide of Claim 1 which has the structure:

a) cPND7 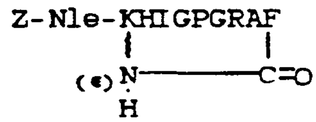 ,

b) cPND8 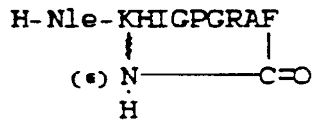 ,

c) cPND10 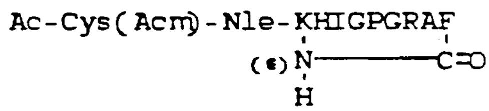 ,

d) cPND9 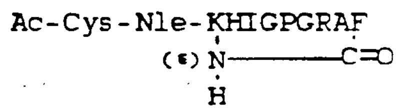 ,

e) cPND11 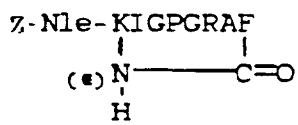 ,

f) cPND12 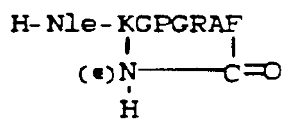 ,

g) cPND13 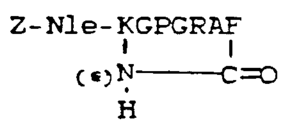 ,

h) cPND14 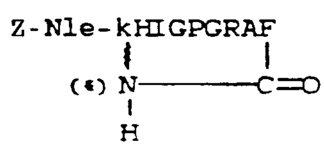 ,

i) cPND15 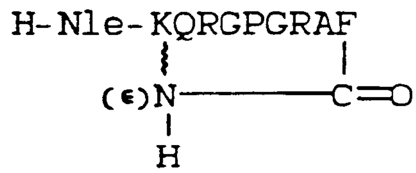 ,

j) cPND16 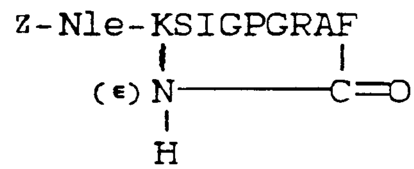 ,

k) cPND21 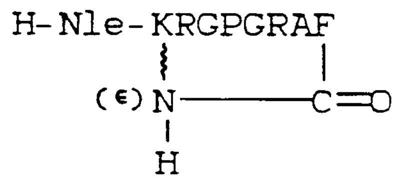 ,

l) cPND23 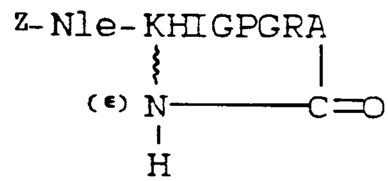 ,

m) cPND24 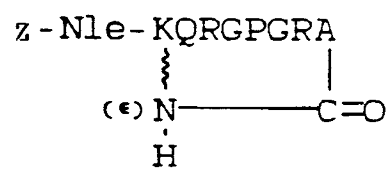 ,

n) cPND25 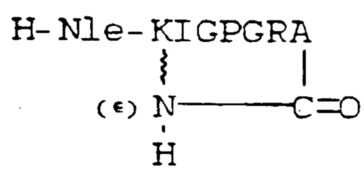 ,

o) cPND26 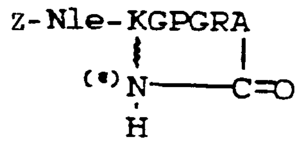 ,

p) cPND27 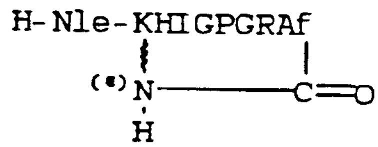 ,

q) cPND28 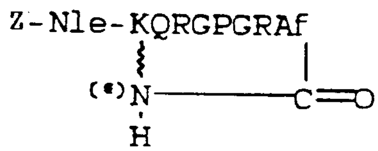 ,

r) cPND29 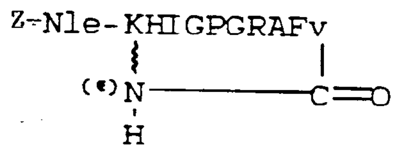 ,

s) cPND30 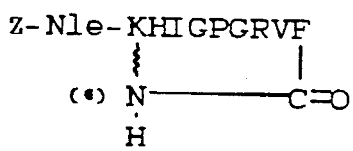 ,

t) cPND31 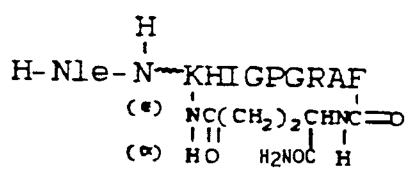 , or

u) cPND32 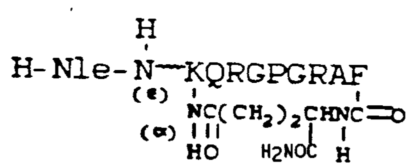 .

**5.** The cyclic peptide of Claim 4 which has the structure:

a) cPND8 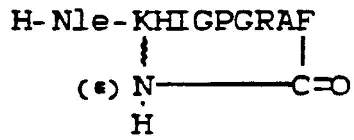 ,

b) cPND18 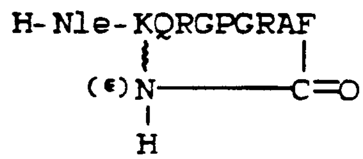 ,

c) cPND31 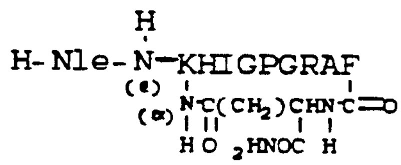 , or

d) cPND32 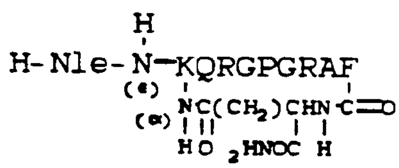 .

6. A process for making the peptide of Claim 1 comprising the steps of (a) synthesizing a linear peptide containing appropriately protected amino acids; (b) cleaving the peptide from the solid support when the peptide is prepared by solid phase synthesis; (c) neutralizing and desalting the cleaved peptide solution; (d) solubilizing the product of step (c) in an organic solvent selected from DMF or $CH_2Cl_2$; and (e) contacting the product of step (d) with a reagent selected from among DPPA and BOP which mediates peptide bond formation between an unprotected amino group on the one side of the loop amino acids, and a free carboxyl group on the other side of the loop amino acids.

7. The process of Claim 6 wherein the free amino group is selected from among the ε-amino group of a lysine, the α-amino group of a lysine, or the peptide's free amino terminus, and the free carboxyl group is selected from the peptide free carboxy terminus or an acidic amino acid side chain, selected from glutamic acid, aspartic acid, isoglutamic acid.

8. The process of Claim 7 wherein the peptide is cyclized by treatment with DPPA in DMF

9. The process of Claim 8 for making a peptide having the structure:

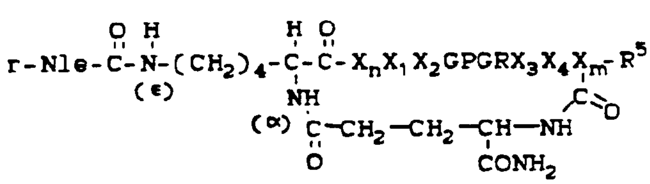

which comprises cyclizing a linear peptide having the structure:

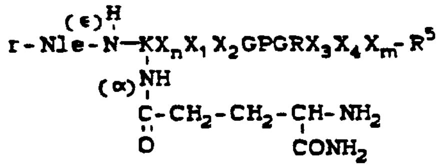

wherein:

the squiggly line represents -($CH_2$)4-;

-GPGR- is the tetramer -GlyProGlyArg-;

$X_1$ is a constituent of $R^2$ selected from:

a) serine,
b) proline,
c) arginine,
d) histidine,
e) glutamine, or
f) threonine;

$X_2$ is a constituent of $R^2$ selected from:

a) isoleucine,
b) arginine,
c) valine, or
d) methionine;

$X_n$ is is a constituent of $R^2$ and is either a bond or a peptide of up to 15 amino acids;

$X_3$ is a constituent of $R^3$ selected from:

a) alanine,
b) arginine, or
c) valine;

$X_4$ is a constituent of $R^3$ and is selected from:

a) phenylalanine,
b) isoleucine,
c) valine, or
d) leucine; and

$X_m$ is a constituent of $R^3$ and is a bond or a peptide of up to 15 amino acids.

**10.** The process of Claim 8 for making a peptide having the structure:

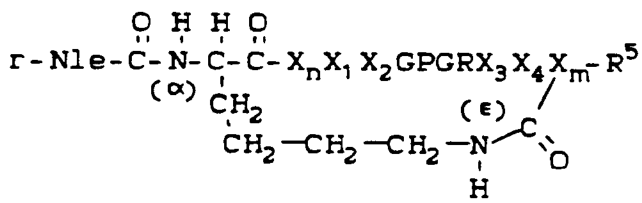

which comprises cyclizing a linear peptide having the structure:

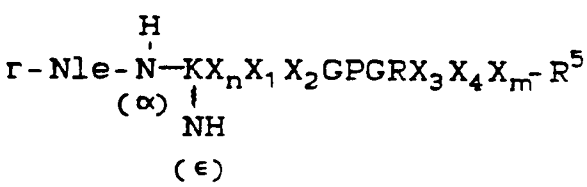

wherein:

the squiggly line represents -$(CH_2)_4$-;

-GPGR- is the tetramer -GlyproGlyArg-;

$X_1$ is a constituent of $R^2$ selected from:

a) serine,

b) proline,
c) arginine,
d) histidine,
e) glutamine, or
f) threonine;

$X_2$ is a constituent of $R^2$ selected from:

a) isoleucine,
b) arginine,
c) valine, or
d) methionine;

$X_n$ is is a constituent of $R^2$ and is either a bond or a peptide of up to 15 amino acids;

$X_3$ is a constituent of $R^3$ selected from:

a) alanine,
b) arginine, or
c) valine;

$X_4$ is a constituent of $R^3$ and is selected from:

a) phenylalanine,
b) isoleucine,
c) valine, or
d) leucine; and

$X_m$ is a constituent of $R^3$ and is a bond or a peptide of up to 15 amino acids.